# EUROPÄISCHE PATENTSCHRIFT

(11) **EP 2 921 565 B1**
(45) Veröffentlichungstag und Bekanntmachung des Hinweises auf die Patenterteilung: **24.07.2019**
(21) Anmeldenummer: 15150399.2
(22) Anmeldetag: 08.01.2015
(51) Int. Cl.: C21D 7/12, C21D 9/08, C22F 1/06, C22F 1/10, C21D 6/00, A61F 2/24, A61L 31/14, A61F 2/00, A61F 2/01, B21D 26/033, A61L 27/50, B05D 3/12

(54) **Thermochemisch behandelte Miniaturrohre als Halbzeuge für vaskulare Stents**
Thermochemically treated miniature tubes as semifinished products for vascular stents
Tuyaux miniatures traités thermochimiquement en tant que semi-produits pour endoprothèses vasculaires

(30) Priorität: 22.01.2014 US 201461929985 P
(43) Veröffentlichungstag der Anmeldung: 23.09.2015
(73) Patentinhaber: Biotronik AG, 8180 Bülach (CH)
(72) Erfinder: Bayer, Ullrich, 18209 Bad Doberan (DE)
(74) Vertreter: Galander, Marcus

(56) Entgegenhaltungen:
- US-A1- 2011 288 630

## Beschreibung

Die Erfindung betrifft ein Verfahren zur Erhöhung der Wechselfestigkeit eines röhrenförmigen Halbzeuges für ein medizinisches Implantat, ein röhrenförmiges Halbzeug für ein medizinisches Implantat mit verbesserter Wechselfestigkeit sowie ein medizinisches Implantat hergestellt aus einem solchen Halbzeug.

### Stand der Technik

Bei mechanisch belasteten Implantaten besteht ein ständiger Bedarf an Lösungen, die zu einer Erhöhung der Lebensdauer des Implantates unter mechanischer Belastung führen. So sind beispielsweise Stents und Herzklappen regelmäßig zyklischen mechanischen Beanspruchungen ausgesetzt. Um bei diesen Bauteilen die mechanische Belastbarkeit (und damit die Lebensdauer) zu erhöhen, werden im Stand der Technik eine oder mehrere der folgenden Maßnahmen durchgeführt:
- Beschichtung von finalen Implantaten mit organischen und anorganischen Schutzschichten zur Erhöhung der Degradationsbeständigkeit (z. B. galvanische Beschichtungen mit Zn, Beschichtungen auf der Basis ionischer Flüssigkeiten, Konversionsschichten durch chemische Umwandlung der Hauptlegierungsbestandteile, Bedampfen oder Sputtern mit Al, thermisches Spritzen....)
- Herstellung von rohrförmigen Halbzeugen aus mehrlagigen Verbundwerkstoffen (auch zur Erhöhung der Röntgensichtbarkeit) durch Plattieren.
- Verwendung von höher legierten Ausgangsmaterialien mit verbesserter Degradationsbeständigkeit
- Verwendung von galliumhaltigen Elektrolyten bei plasmachemischen Oberflächenreaktionen zur Verbesserung der Biokompatibilität (DE 2009/050048) von absorbierbaren Implantaten
- Verwendung von galliumhaltigen Schmierstoffen bei der Hochtemperaturumformung von Mg- Hülsen.
   - Aufbringen von niedrigmoduligen metallischen Beschichtungen zur Erhöhung der Plastifizierbarkeit/Dilatierbarkeit von resorbierbaren Stents.

US 2011/288630 offenbart eine Endoprothese hergestellt aus mehrphasigen eisenhaltigen Stahl. Als Endoprothese kommen dabei Klammern, orthodentische Drähte, Herzklappen oder Stents in Frage.

### Nachteile der Ansätze im Stand der Technik:

Nachträgliche Behandlungsschritte von filigranen Implantaten wie Stents sind aus produktions-technischer Sicht nachteilig, da die zusätzliche Handhabung der Stents in einem weiteren Prozessschritt ein vermehrtes Auftreten von mechanischen Deformationen der Bauteile und damit zu einer erhöhten Ausschussrate verursacht.
Der Vorteil einer erhöhten Degradationsbeständigkeit von höher legierten Ausgangsmaterialien (z. B. Mg mit einem Legierungsanteil von Seltenen Erden ≥ 3%) geht oft mit Nachteilen bei den mechanischen Eigenschaften (geringere Bruchdehnung) einher.

Bei höher legierten Ausgangsmaterialien entstehen bei der Halbzeugfertigung legierungsbedingt Ausscheidungen, die zu lokal inhomogen mechanischen und chemischen Eigenschaftsänderungen führen können. Diese wirken als innere Kerben die wiederum die Entstehung von Anrissen begünstigen.

Konventionell (z. B. mittels Plattierverfahren) hergestellte Halbzeuge zeigen bei der Verformung der daraus gefertigten Endprodukte (z. B. Dilatieren von Stents) häufig Delaminationen der Schicht vom Grundkörper.

Vor dem Hintergrund des Standes der Technik war es Aufgabe der vorliegenden Erfindung, ein Verfahren zur Herstellung von Halbzeugen für Implantate wie Stents oder Herzklappen anzugeben, die sich durch verbesserte Bauteileigenschaften wie Dauerfestigkeit und/oder geringere Rissanfälligkeit auszeichnen.

In bevorzugten Varianten war es ferner wünschenswert, bei degradierbaren metallischen Implantatmaterialien zusätzlich eine Verbesserung der Degradationseigenschaften zu erzielen, wobei bevorzugt auch hier das entsprechende Herstellungsverfahren vereinfacht und/oder beschleunigt sein sollte. Ferner war es für bevorzugte Varianten wünschenswert, eine Erhöhung der Röntgensichtbarkeit der aus den erfindungsgemäß herzustellenden Halbzeugen hergestellten Implantate zu erzielen.

Die oben formulierte Hauptaufgabe wird erfindungsgemäß gelöst durch ein Verfahren zur Erhöhung der Wechselfestigkeit eines röhrenförmigen Halbzeuges für ein medizinisches Implantat, umfassend die Schritte:
a) Bereitstellen des röhrenförmigen Halbzeuges und
b) Beaufschlagen des röhrenförmigen Halbzeuges von innen mit Druck, so dass eine plastische Verformung des Außenumfangs des rohrförmigen Halbzeuges um mindestens 0,2 % erfolgt.

Unter Wechselfestigkeit im Sinne der vorliegenden Erfindung ist die Eigenschaft eines Bauteiles (zum Beispiel eines Halbzeuges bzw. eines daraus entstandenen Implantates) zu verstehen, sich verändernden mechanischen Belastungen zu widerstehen, wobei diese Belastungen bevorzugt zyklisch sind. Die Wechselfestigkeit wird im Zweifelsfall bestimmt, indem man das Bauteil pulsativ (bevorzugt mit 100 Hz) beansprucht und die Zahl der Schwingungen bestimmt, bis zu denen erste Risse entstehen. Diese Zahl der Schwingungen kann bei einem bestimmten konstanten Spannungsniveau ermittelt werden, wobei selbstverständlich je höher das Spannungsniveau ist, desto kleiner ist die Zahl der ermittelten Schwingungen (Lastwechselzahl), ab denen erste Schädigungen auftreten. Von einer Erhöhung der Wechselfestigkeit im Sinne dieses Textes ist dann zu sprechen, wenn für ein gleiches Bauteil vor Durchführung des erfindungsgemäßen Verfahrens eine geringere Lastwechselzahl bestimmt wird als für ein Bauteil nach Durchführung des erfindungsgemäßen Verfahrens, wobei das Bauteil vor Durchführung des erfindungsgemäßen Verfahrens dem Vergleichsbauteil gleich war.

Die Dehngrenze Rp 0,2 stellt diejenige Spannung dar, bei der eine bleibende Dehnung von 0,2 % erreicht wird. Diese Kenngröße wird nach ISO 6892-1bzw. DIN EN ISO 6892-1 im Zugversuch ermittelt. Die Belastungsverhältnisse durch die Innendruckbeaufschlagung von Rohren werden durch den Berstdruckversuch ermittelt. Dieser wird nach DIN ISO 2758 durchgeführt. Dabei wird die Materialprobe kreisförmig auf einer Membran eingespannt, welche sich unter Druckeinwirkung wölbt und die Materialprobe zum Bersten bringt. Der beim Berstvorgang auf die Membran wirkende hydraulische Druck ist zugleich der Berstdruck des zu untersuchenden Materials.

Erfindungsgemäß wird aber der Innendruck nicht so weit erhöht dass es zum Bersten des Rohres kommt. Der Innendruck wird nur solange gesteigert bis eine plastische Verformung des Rohres eintritt die eine bleibende Durchmesser- bzw. Umfangsvergrößerung von mindestens 0,2% der Ausgangswerte hervorruft. So vergrößert sich beispielsweise der Umfang eines Rohres mit dem Ausgangsaußendurchmesser von 2,000 mm bei Erreichen der auf die Rohrgeometrie bezogenen 0,2% Dehngrenze auf 2,004 mm. Das entspricht einer Umfangsvergrößerung von 6,283 mm auf 6,296 mm. Das sind jeweils die Mindestwerte der zu realisierenden plastischen Verformungen. Die dabei zur Anwendung kommenden Innendruckwerte sind in ihrer Höhe werkstoffspezifisch, d.h. sie sind von der Festigkeit des Ausgangsmaterials abhängig.

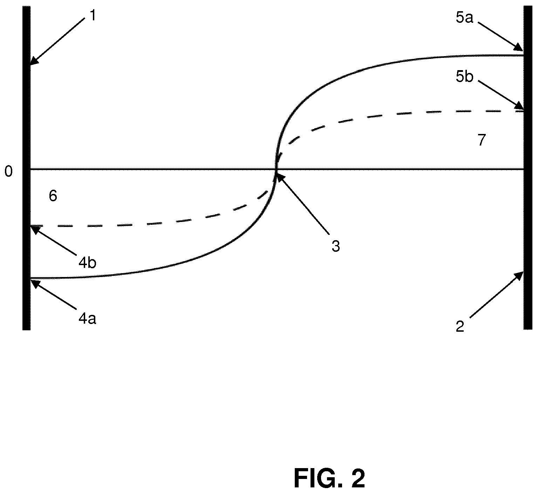

Durch die Durchführung von Schritt b) des erfindungsgemäßen Verfahrens werden über den Rohrquerschnitt entstehende Eigenspannungsverläufe erzeugt. Diese zeichnen sich einerseits durch Druckspannungen auf der Rohrinnenseite und andererseits Zugspannungen auf der Rohraußenseite aus. In nachfolgenden Bearbeitungsschritten wie zum Beispiel bei der Stentherstellung aus dem röhrenförmigen Halbzeug können zwar die Spannungsgradienten verringert werden, sie werden aber nicht vollständig abgebaut, so dass die verbleibenden Druckeigenspannungen die Wechselfestigkeit auch der Endprodukte verbessern, die aus dem erfindungsgemäßen hergestellten Halbzeug hergestellt werden. Dies betrifft insbesondere die Erhöhung der Dauerfestigkeit bei zyklischen Beanspruchungen, die Verringerung der Neigung zur Ausbreitung von Rissen in unter Druckeigenspannungen stehenden Zonen und führt außerdem zu größeren realisierbaren Dilatationsdurchmessern sowie zur Lebensdauererhöhung insbesondere bei nicht degradierbaren Implantatmaterialien wie zum Beispiel der Co-Cr-Legierung L-605 oder auch der Stahllegierung 316L. Erfreulicherweise lässt sich ein entsprechender Eigenspannungsgradient, der die Erhöhung der Wechselfestigkeit bedingt, auch in degradierbaren Materialien wie zum Beispiel Magnesiumlegierungen insbesondere wie WE43 und Z10 erreichen.
Durch die Druckbeaufschlagung auf der Rohrinnenseite als zusätzlicher Schritt in der Halbzeugherstellung entstehen wie beschrieben auf der Rohrinnenseite Druckeigenspannungen, deren Niveau sich bis zur Rohrmitte auf null absenkt. Ab der Rohrmitte in Richtung der Rohraußenseiten entstehen dagegen Zugeigenspannungen. Diese haben ihr Maximum direkt an der Rohraußenseite. Die Eigenspannungen zeigen einen sinusartigen Verlauf (vgl. Fig. 2).

Der Grundgedanke der Erfindung besteht, vereinfacht ausgedrückt, darin, das rohrförmige Halbzeug temporär von innen derart mit Druck zu beaufschlagen, dass die Dehngrenze Rp 0,2 des Materials überschritten wird und eine Eigenspannung im Material entsteht.

Die Innendruckverformung findet bei Drücken statt, die das jeweilige Rohr über die Dehngrenze Rp 0,2 hinaus plastisch verformen. Dabei tritt bevorzugt eine (Innen-)Durchmesserveränderung (Vergrößerung) im Bereich von 0,5 % bis 1,5 % ein.

Bevorzugte Größen für die in das erfindungsgemäße Verfahren einzusetzenden röhrenförmigen Halbzeuge sind:
Außendurchmesser 1-5 mm, bevorzugt 1,2 bis 4 mm, weiter bevorzugt 1,5 bis 3 mm und besonders bevorzugt 1,8 bis 3,5 mm und/oder
Wandstärke 0,01 bis 1 mm, bevorzugt 0,05 bis 0,5 mm und weiter bevorzugt 0,07 bis 0,4 mm und/oder
Längen von 10 bis 5000 mm, bevorzugt 50 bis 1500 mm und besonders bevorzugt 100 bis 2000 mm.

Grundsätzlich kann das in das erfindungsgemäße Verfahren einzusetzende Halbzeug aus verschiedenen Grundmaterialien wie z.B. Kunststoff, und hier insbesondere Polylactid bestehen.

Bevorzugt besteht das röhrenförmige Halbzeug jedoch aus Metall oder einer Metalllegierung.

Bevorzugte Legierungen sind:
Auf dem Gebiet der resorbierbaren Magnesiumlegierungen:
- WE43
- AZ31
- AZ61
- ZK10

Alle diese Legierungen können bei Bedarf bevorzugt jeweils mit bis zu 1% Ca auflegiert werden.

Auf dem Gebiet der nicht resorbierbaren Legierungen:
Stahl 316 L (rost und säurebeständiger Stahl mit ca. 16,5-18,5% Cr; 10,5-13,5 % Ni; 2,0 - 2,5% Mo; max. 2,0% Mn; max. 1,0% Si; max. 0,07% C; Rest Fe)
Cobalt-Basislegierung L-605 (19-21 % Cr; 14-16 % W; 9-11% Ni; 1-2% Mn; max 3 % Fe Rest Co)
Cobalt-Nickellegierung MP35N (35% Ni; 20% Cr; 10 %Mo)
Nitinol (Legierung aus Ni + Ti) (54,5% -57%Ni; Rest Ti)

Bei metallischen Materialien lassen sich die durch das erfindungsgemäße Verfahren erzielbaren Effekte besonders gut erreichen. Dazu gehört auch, dass neben der Erhöhung der Wechselfestigkeit eine Verringerung der Kerbempfindlichkeit durch das erfindungsgemäße Verfahren entsteht. So wird durch das Aufbringen von Druckeigenspannungen in die Innenoberflächen des röhrenförmigen Halbzeugs der Aufbau von kerbbedingten Spannungsfeldern in die Rohrwanddicke erheblich verringert.

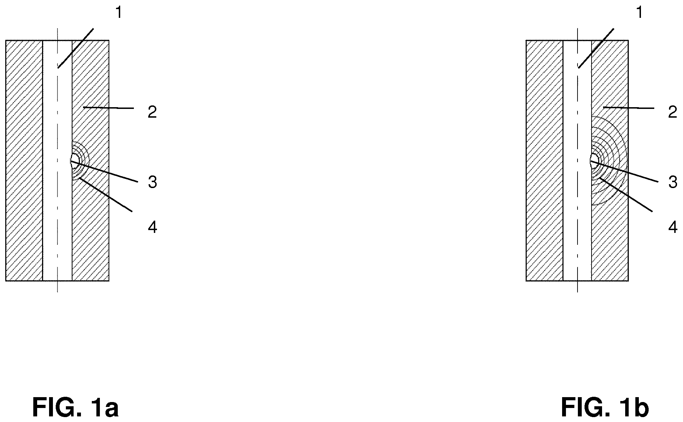

Dies ist schematisch in der Fig. 1 dargestellt, wobei die Fig. 1a ein durch ein erfindungsgemäßes Verfahren hergestelltes röhrenförmiges Halbzeug darstellt und die Fig. 1b ein Halbzeug darstellt, das dem erfindungsgemäßen Verfahren nicht unterworfen wurde. Die Bezugszeichen bedeuten dabei folgendes:
1 Lumen
2 Röhrenwand
3 Kerbung der Innenwand
4 Aufgrund der Kerbung entstandene Spannungen

Wie aus dem schematischen Vergleich deutlich erkennbar, sind die Kerbspannungen in der Fig. 1b (nicht durch das erfindungsgemäße Verfahren hergestelltes röhrenförmiges Halbzeug) deutlich höher.

Mit dem beschriebenen Phänomen einher geht auch, dass auch eine erhöhte Schadenstoleranz beim Prozessieren des Halbzeugs zum fertigen Endprodukt besteht. Gleichzeitig besteht eine erhöhte Prozesssicherheit, da eine geringere Bruchwahrscheinlichkeit beim Dilatieren durch das erfindungsgemäße Verfahren erzeugt wird.

Erfindungsgemäß bevorzugt ist ein Verfahren, wobei das Halbzeug ein Halbzeug für einen Stent oder für eine Herzklappe ist.

Wie bereits oben angedeutet, lassen sich diese Endprodukte durch das erfindungsgemäße Verfahren für das Halbzeug in einer besonders hohen Qualität herstellen.

Mit der Druckbeaufschlagung des röhrenförmigen Halbzeuges von innen ist ein weiterer Effekt erzielbar: Es ist möglich, durch eine entsprechende Druckbeaufschlagung mit geeigneten Medien, bevorzugt Flüssigkeiten, eine Auflegierung der Rohrinnenoberflächen oder eine Auflegierung von Elementen oder Verbindungen in die Rohrwand zu erzielen. Dabei ist es nicht unbedingt erforderlich, dass die Druckbeaufschlagung so erfolgt, dass eine Verformung des Halbzeuges bis oberhalb der Dehngrenze Rp 0,2 erfolgt. Letzteres ist aber eine bevorzugte Variante und somit mit dem erfindungsgemäßen Verfahren gut kombinierbar.

Dementsprechend ist es auch in einem erfindungsgemäßen Verfahren bevorzugt, dass die Druckbeaufschlagung mittels einer Flüssigkeit erfolgt. Dies kann auch bevorzugt sein, wenn es nicht zur Auflegierung von Anteilen der Flüssigkeit in die Oberfläche des Halbzeugs kommt, besonders bevorzugt ist aber die Variante, wobei es zur Auflegierung von Anteilen der Flüssigkeit zur Druckbeaufschlagung in die Innenoberfläche des Halbzeuges kommt.

Eine "Auflegierung" im Sinne des vorliegenden Textes liegt dann vor, wenn sich die Legierungszusammensetzung nach dem Vorgang der "Auflegierung" verändert hat.

Diese Auflegierung bewirkt eine graduelle Veränderung der chemischen Zusammensetzung über die Rohrwand. Das bedeutet, dass die Rohrinnenwand die stärkste Veränderung der chemischen Zusammensetzung erfährt, da diese direkt mit der Flüssigkeit, beispielsweise schmelzflüssiges Metall oder eine Legierung, des Druckmediums in Kontakt kommt. Die Rohrmitte und Rohraußenwand bleiben dagegen in ihrer chemischen Zusammensetzung unverändert.

Die Druckmedien variieren je nach den einzustellenden Eigenschaftsprofilen. Dies kann im Falle der Anwendung von L-605 Wasser sein. Wenn dieses mit einem Druck von z. B. ca. 1.500 bar in das Rohr gedrückt wird, entsteht ein Eigenspannungsprofil mit Druckeigenspannungen auf den Innenoberflächen und Zugeigenspannungen auf den Rohraußenoberflächen. Die Druckeigenspannungen generieren eine höhere Wechselfestigkeit und eine geringere Kerbempfindlichkeit der daraus gefertigten Stents wenn diese dilatiert und anschließend zyklisch beansprucht werden.

Bei den resorbierbaren Materialien ist bevorzugt, dass durch die Druckbeaufschlagung eine (relativ niedrig schmelzende) CaZn- Legierung (besonders bevorzugt eine CaZn-Legierung mit 27 Masse-% Zn) einlegiert wird. Dieses ist möglich bei Temperaturen ≤ 420°C sowohl in Magnesiumoberflächen als auch in die Oberflächen von röhrenförmigen Halbzeugen aus korrodierenden Eisenbasislegierungen oder Reineisen. Durch Variation des Druckes, der Verweilzeit des Mediums im Rohrinneren und der Temperatur gelingt mit derartigen thermochemischen Verfahren die Herstellung neuartiger, mechanisch und chemischfunktioneller Halbzeugen, insbesondere für Stents.

Durch die Verwendung von geeigneten flüssigen oder gasförmigen Trägern als Medien bei der Innendruckumformung werden Diffusionseffekte generiert, die zu einer Änderung der Innenoberflächenzusammensetzung der Rohre führen. Die dabei erreichbare Tiefe, in der die chemische Zusammensetzung der Wanddicke verändert wird, kann durch Variation der Temperatur, der Zeit, des konkret anliegenden Druckverlaufs und des Mediums in weiten Grenzen variiert werden.

Durch das Einlegieren aufgrund der Innendruckbeaufschlagung auf die Innenwand des röhrenförmigen Halbzeuges lassen sich (insbesondere im erfindungsgemäßen Verfahren) eine Vielzahl von positiven Effekten, die teilweise gleichzeitig erwünscht sind, erzielen:
- Auflegierung der Rohrinnenoberflächen mit Metallen und Legierungen, die einen funktionellen Einfluss auf die Degradationsverläufe und die Biokompatibilität ausüben. Diese sind z. B. magnesiumhaltige Salze wie z. B. Magnesiumnitrat, die eine Beschleunigung der Degradation von Fe bewirken.
- Bei degradierbaren metallischen Implantatmaterialien wird sowohl eine Verbesserung der mechanischen Eigenschaften (Dauerfestigkeit und geringere Rissanfälligkeit) als auch eine Verbesserung der Degradationseigenschaften durch Auflegieren der Stentinnenoberflächen erzielt.
- Bei degradierbaren Materialien wird durch Anwendung der Innendruckbeaufschlagung mittels schmelzflüssiger Phasen neben der Auflegierung der Innenoberfläche gleichzeitig die Einstellung definierter mechanischer Eigenschaften über den Rekristallisationsgrad (Temperatur, Haltezeit) möglich.
- Bei degradierbaren Materialien bewirkt das Einlegieren von Legierungselementen unter hohen Drücken eine wesentliche Beschleunigung der ansonsten nur zeit- und temperaturgesteuerten Diffusion. Dies führt zu einem wesentlich effektiveren Prozess der Oberflächenvergütung.
- Durch Variation der Verfahrensparameter (Temperatur, Zeit) ergibt sich ein großes Spektrum an einstellbaren Diffusionstiefen von Legierungselementen in das Grundmaterial hinein. Dadurch können bei degradierbaren Materialien zeitlich variable Degradationsfenster vorgegeben werden.
- Eine Beschleunigung der Degradation bei Fe-Basislegierungen ist mittels lokalelementbildender Edelmetallsalze wie z. B. Silberchlorid (Ts= 455°C), Silberbromid (Ts= 435°C), Silberjodid (Ts= 552°C) möglich. Bei diesen Temperaturen findet sowohl eine Diffusion der Einzelelemente als auch der Verbindung selbst in den Fe-bulk statt. Dies führt zu einer beschleunigten Degradation des daraus gefertigten Bauteils.
- Es ergeben sich Möglichkeiten der Verbesserung der Biokompatibilität von Stents durch Aufbringen von höher kompatiblen Werkstoffen in die Oberflächen von geringer biokompatiblen Basismaterialien.
- Es besteht die Möglichkeit der Einstellung von Unterschieden in der chemischen Zusammensetzung von luminarer und abluminarer Oberfläche.
- Eine verzögerte Degradation der aus dem Halbzeug gefertigten Implantate (Verschiebung des elektrochemischen Potentials) kann erreicht werden.
- Durch die Anwendung von eutektischen Legierungen als Umformmedium bei der Innendruckverformung erweitern sich wesentlich die Möglichkeiten der chemischen Oberflächenfunktionalisierung durch Eindiffusion von mehreren Elementen in die Oberflächen des Basismaterials.
- Die Verwendung von chloridhaltigen Salzen (z. B. MgCl, NaCl), oder Magnesiumnitrat bzw. Magnesiumnitrat-Hexahydrat oder Citronensäure, die eine Oberflächenaufrauung von Fe bewirken, lässt sich eine Verringerung der Korrosionsbeständigkeit hervorrufen.
- Zink- und calziumhaltige Verbindungen und/oder phosphathaltige Verbindungen (z. B. Dikaliumhydrogenphosphat) bewirken während des Prozesses Diffusionseffekte im Magnesium die zur Erhöhung der Korrosionsfestigkeit führt (Bildung von oberflächennahem Magnesiumphosphat).
- Schmelzflüssige Legierungen oder Mischungen aus Härtesalzen erlauben die Verwendung dieser sowohl als Medium für das Innenhochdruckverfahren zur Einbringung der Eigenspannungen als auch (bzw. gleichzeitig) als Medium für die einzustellenden Materialdiffusionseffekte.
- Durch das Oberflächenlegieren von Implantatmaterialien mit Metallen die einen geringeren E-Modul als das Basismaterial aufweisen (z. B. Zn mit einem E-Modul von 85 GPa im Vergleich zu Fe mit 210 GPa und L-605 mit 243 GPa), verringern sich die Risiken der frühzeitigen Entstehung und Weiterverbreitung von Anrissen
- Es ergeben sich Möglichkeiten der gleichzeitigen Erhöhung der Röntgensichtbarkeit und der Biokompatibilität durch den Einsatz von schmelzflüssigen eutektikumsnahen binären Legierungen (z. B. Pt-Ca)

Dementsprechend ist es bevorzugt (insbesondere in dem erfindungsgemäßen Verfahren), dass die Druckbeaufschlagung insbesondere von Rohren aus bioresorbierbaren Magnesiumlegierungen mittels niedrigschmelzender Metalle oder Metalllegierungen (Ts ≤ 420°C), bevorzugt flüssiges Sn, Zn, In, Ga, Li, Na, Bi und deren Legierungen erfolgt.

In diesen Metallen können eine Vielzahl der oben beschriebenen Effekte durch den Fachmann erreicht werden.

Dabei ist es bevorzugt, dass eine Auflegierung bis maximal 50 % der Tiefe des Halbzeugquerschnittes erfolgt.

Dies kann der Fachmann durch entsprechende Druckbeaufschlagung (Druck, Dauer, Temperatur) gut steuern.

Grundsätzlich ist es möglich, durch die Druckbeaufschlagung (insbesondere im erfindungsgemäßen Verfahren) die Diffusion von Elementen in die Wand des röhrenförmigen Halbzeuges zu bewirken, die zu einer Verringerung der Degradationsgeschwindigkeit von resorbierbaren Materialien, insbesondere Mg-Legierungen führen.

Resorbierbar im Sinne dieser Beschreibung sind dabei Materialien, die im menschlichen Körper ohne Schädigung desselben abgebaut werden können oder in Verbindungen umgewandelt werden die den Körper ebenfalls nicht schädigen.

Ebenso ist es möglich, durch die Druckbeaufschlagung (insbesondere im erfindungsgemäßen Verfahren) eine Auflegierung von Elementen zur Beschleunigung der Degradationsgeschwindigkeit von resorbierbaren Legierungen, hier insbesondere Fe-Legierungen, zu bewirken.

Außerdem ist es möglich (insbesondere auch im erfindungsgemäßen Verfahren), eine Kombination aus Verbesserung der mechanischen Eigenschaften und der biologisch funktionellen Eigenschaften zu erzielen, indem in Abhängigkeit des Basismaterials entsprechende Elemente zur Verringerung der Degradationsgeschwindigkeit (insbesondere bei resorbierbaren Magnesiumlegierungen) und solche zur Beschleunigung der Degradationsgeschwindigkeit insbesondere bei resorbierbaren Eisenlegierungen) durch Druckbeaufschlagung in die Wände des Halbzeuges einlegiert werden. So ist es zum Beispiel möglich, bei aus diesen Halbzeugen hergestellten Implantaten (insbesondere Stents) die Einhaltung zielgenauer Degradationskorridore zu gewährleisten, wobei durch das erfindungsgemäße Verfahren parallel die Verbesserung der Wechselfestigkeit und außerdem auch die Verbesserung der Plastifizierbarkeit (was einen größeren Dilatationsdurchmesser ermöglicht (insbesondere für Stents) zu gewährleisten.

Entsprechend dem oben Gesagten ist ein Teil der Erfindung ein röhrenförmiges Halbzeug für ein medizinisches Implantat, wobei an der Innenoberfläche Druckeigenspannungen vorliegen, die ca. 15 % bevorzugt 20 % weiter bevorzugt 30 % und besonders bevorzugt 40 % der bei Raumtemperatur im Zugversuch ermittelten Dehngrenze Rp 0,2 betragen und/oder wobei Druckeigenspannungen von ≥ 20 MPa. Bevorzugt liegt an der Innenoberfläche eine Druckeigenspannung von ≥ 30 MPa, weiter bevorzugt ≥ 50 MPa und besonders bevorzugt ≥ 75 vor.

Zwar werden die Eigenspannungen nach dem Laserschneiden und dem Elektropolieren verringert, sie gehen aber nicht auf Null zurück. So verbleiben noch ca. 40 bis 50 % der nach der Druckbeaufschlagung vorliegenden Eigenspannungen im finalen Bauteil zurück. So geht man beispielsweise davon aus, dass ein Rohr aus einer bioresorbierbaren Magnesiumlegierung wie WE 43 im unbehandelten Zustand eine Dehngrenze Rp 0,2 z. B. von 200 MPa aufweist. Ein innendruckbeaufschlagtes Rohr derselben Legierung hat auf der Rohrinnenseite Druckeigenspannungen von 80 MPa (= 40 % der Dehngrenze). Nach dem Laserschneiden und der Elektropolitur verringern sich diese auf ca. 40 MPa (=50 % der ursprünglich vorhandenen Eigenspannungen). Diese reichen aber noch aus für die genannten Eigenschaftsverbesserungen. Die Dehngrenze Rp 0,2 wird dabei im Zugversuch nach DIN EN ISO 6892-1 ermittelt

Die Druckeigenspannungen an der Innenseite bzw. die inversen Zugeigenspannungen auf der Außenseite des erfindungsgemäßen röhrenförmigen Halbzeuges lassen sich mittels röntgenographischer Eigenspannungsanalyse ermitteln. Bevorzugt werden dabei Diffraktions- oder Beugungsverfahren angewendet, wie z.B. hochauflösende Röntgendiffraktometrie (HRXRD) mit dem das Gerät inel EQUINOX 6000 des Unternehmens inel Inc, aus Stratham, 03885 NH, USA oder das Röntgendiffraktometer D 5000 von Siemens.

Dabei werden eigenspannungsbedingte Deformationen in submikroskopischen Bereichen als Gitterdehnung kristalliner Werkstoffe (wie die genannten Legierungen für die erfindungsgemäßen Implantate) ermittelt. Aus der gemessenen Gitterdehnung wird über elastizitätstheoretische Beziehungen der vorliegende Eigenspannungszustand bestimmt. Zur Messung der Gitterdehnung kommen Röntgenstrahlen zum Einsatz. Der Vorteil dieser zur Anwendung kommenden Messmethode liegt einerseits in der Zerstörungsfreiheit, andererseits in der hohen Messgenauigkeit von +/- 1 MPa. Zudem ist diese Methode gegenüber den alternativ zum Einsatz kommenden Neutronenstrahlverfahren weniger aufwändig. Allerdings beschränkt die geringe Eindringtiefe der Röntgenstrahlen in metallische Bauteile deren Einsatz auf oberflächennahe Bereiche von wenigen µm. Um Eigenspannungen in größeren Bauteiltiefen zu ermitteln, kommen schichtweise Abtragsverfahren zum Einsatz. Bei den Implantmaterialien und den daraus gefertigten Stents sind dies vorzugsweise elektrochemische Verfahren wie das Elektropolieren. Diese Methode stellt sicher, dass keine zusätzlichen Verformungen in das Material eingebracht werden, die wiederum zu verfälschten Eigenspannungszuständen führen würden. Bei Magnesiumlegierungen wird in phosphorsäurehaltigen Elektrolytzusammensetzungen bei Anlegen einer anodischen Spannung von ca. 6 V an die Bauteile eine Abtragsrate von ca. 10 µm/min erreicht. Durch Anwendung dieser Methode kann der jeweilige Eigenspannungszustand ermittelt werden. Allerdings muss beachtet werden, dass trotz dieses behutsam durchgeführten Materialabtrages eine Beeinflussung des Kräfte- und Momentengleichgewichts im Eigenspannungsfeld nicht ganz auszuschließen ist. Deshalb sind rechnerische Korrekturen vonnöten die die den Eigenspannungszustand an der neu entstandenen Oberfläche im Vergleich zum Bauteil vor dem Abtrag bestimmen. Mittels Anwendung von Finite Elemente Methoden (FEM) können diese in Abhängigkeit vom abgetragenen Materialvolumen und der jeweils vorliegenden Bauteilgeometrie kalkuliert werden. Allerdings erhöht sich die Messungenauigkeit auf ca. +/- 5 MPa.

Wie bereits oben beschrieben sind die erfindungsgemäßen röhrenförmigen Halbzeuge aufgrund ihrer Eigenspannungen besonders widerstandsfähig gegenüber mechanischen Beanspruchungen. Bevorzugt ist ein erfindungsgemäßes röhrenförmiges Halbzeug für ein medizinisches Implantat, umfassend eine auflegierte Innenseite.

Dabei ist diese Auflegierung über eine Druckbeaufschlagung im Rahmen eines erfindungsgemäßen Verfahrens erzielbar. Dieses lässt sich durch eine Gradientenbildung feststellen, wobei der Grad der Auflegierung durch metallographische Querschliffe bestimmt werden kann, die im Rasterelektronenmikroskop mittels energiedispersiver Röntgenanalyse hinsichtlich ihrer Materialzusammensetzung ortsaufgelöst gescannt werden. Das entstehende Elementmapping zeigt die über den Rohrwandquerschnitt verlaufenden Gradienten in der chemischen Zusammensetzung.

Entsprechend dem oben Gesagten ist ein bevorzugtes erfindungsgemäßes röhrenförmiges Halbzeug für ein medizinisches Implantat ein solches, das hergestellt ist nach einem erfindungsgemäßen Verfahren.

Teil der Erfindung ist auch ein medizinisches Implantat, hergestellt aus einem erfindungsgemäßen Halbzeug. Bevorzugt ist dabei das medizinische Implantat ein Stent oder eine Herzklappe.

Wie bereits weiter oben beschrieben ist es möglich, eine ausreichend starke Eigenspannung aus dem erfindungsgemäßen röhrenförmigen Halbzeug bei der Verarbeitung zu den erfindungsgemäßen (insbesondere den bevorzugten erfindungsgemäßen) Implantaten zu erhalten. Somit ist es möglich, auch diesen Implantaten eine verbesserte mechanische Haltbarkeit zu vermitteln.

Dass ein ausreichender Spannungsgradient bzw. ein Eigenspannungsniveau von ca. 50 % des ursprünglichen Eigenspannungsniveaus des innendruckbeaufschlagten Rohres auch nach dem Laserschneiden des Rohres erhalten bleibt, kann bevorzugt durch eine oder mehrere der folgenden Maßnahmen sichergestellt werden:
- Einsatz von Ultrakurzzeitlasern (z.B. Femtosekundenlaser) die nur einen minimalen Wärmeeintrag in die Rohrwand hervorrufen und damit nur eine minimale Wärmeeinflusszone an der Laserschnittkante generieren.
- Sehr geringe Materialabträge bei den nachfolgenden Beiz- und Elektropolierprozessen. Die bedeutet beispielhaft, dass eine ursprüngliche Rohrwanddicke von 90 µm nur auf 80 µm abgetragen wird und eine nach dem Laserschneiden vorhandene Stegbreite von 100 µm am finalen elektropolierten Stent noch mindestens 90 µm aufweisen muss.

Unabhängig davon oder sogar zusätzlich hierzu lassen sich auf die erfindungsgemäßen Implantate die oben beschriebenen Vorteile aufgrund von Auflegierungen mittels Druckbeaufschlagung der Innenwand des röhrenförmigen Halbproduktes übertragen.

### Beispiele

### Ausführungsbeispiele der erfindungsgemäßen Lösung

Im Folgenden werden für einige ausgewählte rohrförmige Halbzeuge beispielhafte Druckwerte und Beaufschlagungszeiten gegeben, bevor dann drei Ausführungsbeispiele der Erfindung konkret beschrieben werden.

| Werkstoff | Druck - medium | Rp0,2 Dehngrenze des Ausgangsrohres [MPa] | Zu beaufschlagender Innendruck [bar] | Anfangstemperatur des Druckmediums [°C] | Haltezeit bei Maximaldruck [s] |
|---|---|---|---|---|---|
| Implantatstahl 316L | Wasser | 700 | 1000 - 1500, bevorzugt 1100 | RT | 5-30, bevorzugt 10-20, besonders bevorzugt 15 |
| Co-Basislegierung L-605 | Wasser | 600 | 800-1400, bevorzugt 1000 | RT | 5-30, bevorzugt 10-20, besonders bevorzugt 15 |
| Magnesiumlegierung WE 43 | Ca-Zn-Legierung (73% Ca, 27% Zn) | 200 | 100 - 200, bevorzugt 150 | 380 - 450, bevorzugt 410 | 600-1200, bevorzugt 700-1000, besonders bevorzugt 800 |
| Reineisen | Magnesiu mnitrat-Hexahydrat Mg(NO₃)₂ ·6H₂O | 200 | 150 - 200, bevorzugt 175 | 80 - 120, bevorzugt 100 | 600-1200 bevorzugt 700-1000 besonders bevorzugt 800 |

### 1.) L-605 (Rohr aus CoCr-Legierung)

Ein Rohr einer CoCr- Legierung weist folgende Legierungszusammensetzung (Gewichtsprozent) auf:
19-21 % Cr
14-16 % W
9-11% Ni
1-2% Mn
max 3 % Fe
Rest Co

Dieses Rohr hat eine Länge von 2 m und einen Außendurchmesser von 2,00 mm bei einer Wanddicke von 0,10 mm. Die Rp 0,2-Dehngrenze des Ausgangsrohres beträgt 600 MPa. Durch eine Innendruckbeaufschlagung mit Wasser von ursprünglich 20 °C bei 1.000 bar wird dieses über einen Zeitraum von 15 s kurz über der Dehngrenze plastisch verformt. Der Außendurchmesser vergrößert sich dabei auf 2,01 bis 2,02 mm. Dies bedeutet, dass das Material oberhalb der Rp 0,2 Dehngrenze verformt wurde. Das heißt, die bleibende Dehnung beträgt ca. 1 %. Die dabei direkt an der Innenrohroberfläche entstehenden Druckeigenspannungen erreichen Werte von 500 MPa. Die Zugeigenspannungen an der Rohraußenoberfläche erreichen 500 MPa (vgl. Fig. 2).

Beim nachfolgenden Laserschneiden und den Beiz- und Elektropolierprozessen in mineralischen Säuregemischen werden die Druck- und Zugeigenspannungen auf ca. 50 % abgebaut. Der finale Stent hat nunmehr einen Außendurchmesserbereich zwischen 2,00 und 2,01 mm. Das bedeutet, dass von den beiden Rohrwandseiten jeweils 5 µm innen und 5 µm außen abgetragen wurden. In der Summe verringert sich der Außendurchmesser um 10 µm und der Innendurchmesser vergrößert sich um 10 µm gegenüber dem innendruckbeaufschlagtem Rohr. Die verbleibenden Druckeigenspannungen der Innenoberflächen des nunmehr vorhandenen Stents betragen nunmehr noch zwischen 200 und 250 MPa. Die daraus resultierende erhöhte Wechselfestigkeit erhöht die Grenzlastspielzahl um 25 % gegenüber Stents aus unbehandeltem Rohr.

### 2.) Rohre aus der Mg-Legierung WE 43

Diese Legierung besteht aus 4 Gew.-% Y und 3 Gew.-% Seltenen Erden (Nd, Dy, Gd) sowie ca. 0,5 Gew.-% Zr, Rest Mg.
Dieses Rohr hat eine Länge von 2 m und einen Außendurchmesser von 2,00 mm bei einer Wanddicke von 0,18 mm. Die Rp 0,2-Dehngrenze des Ausgangsrohres beträgt 200 MPa. Eine schmelzflüssige Legierung aus 27 % Zn und 73 % Ca wird bei 410°C mit einem Druck von 150 bar in ein Mg-Rohr gepresst. Die Dauer der Druckbeaufschlagung des Rohres mit dem schmelzflüssigen Medium beträgt 10 bis 20 min. Durch Diffusionseffekte wird die Innenoberfläche mit Ca und Zn bis in eine Tiefe von 30 µm auflegiert. Dadurch vergrößert sich die Wanddicke von 180 µm auf nunmehr 190 µm. Von den 30 µm der auflegierten Rohrinnenwand werden bei der Stentfertigung ca. 10 µm wieder durch Beizen und Polieren abgetragen. Der Abtrag der nicht auflegierten Außenwand beträgt ebenfalls ca. 10 µm. Damit verbleibt eine Stentwanddicke von 170 µm. Das an der Stentinnenseite verbleibende Druckeigenspannungsniveau liegt im Bereich von 80 MPa.

Der finale fertig prozessierte Stent hat einen Außendurchmesser von 1,98 mm Die verbleibenden 20 µm dicke auflegierte Zone der Rohrinnenwand führt zu einer um ca. 4 Wochen verlängerten Degradationszeit im Vergleich zu einem Stent aus unbehandeltem Ausgangsmaterial. Diese Behandlung führt auch zu einer Erhöhung des maximalen Dilatationsdurchmessers um 0,3 mm auf nunmehr 4,8 mm bis es zum ersten Stegbruch kommt. Stents die keine derartige Behandlung erfahren haben zeigen dagegen erste Stegbrüche schon bei 4,5 mm.

### 3.) Cobalt Chromlegierung MP 35 N

Ein Rohr aus einer CoNi- Legierung weist folgende Legierungszusammensetzung (Gewichts-prozent) auf:
34-36 % Ni
18-21 % Cr
9-11 % Mo
Rest Co

Dieses Rohr hat eine Länge von 2 m und einen Außendurchmesser von 1,80 mm bei einer Wanddicke von 0,09 mm. Die Rp 0,2-Dehngrenze des Ausgangsrohres beträgt 1000 MPa. Durch eine Innendruckbeaufschlagung mit Wasser von ursprünglich 20 °C bei 1.300 bar wird dieses über einen Zeitraum von nur 10 s kurz über der Dehngrenze plastisch verformt. Der Außendurchmesser vergrößert sich dabei auf 1,81 bis 1,82 mm. Dies bedeutet, dass das Material kurz oberhalb der Rp 0,2-Dehngrenze verformt wurde. Das heißt, die bleibende Dehnung beträgt ca. 1 %. Die dabei direkt an der Innenrohroberfläche entstehenden Druckeigenspannungen erreichen Werte von 600 MPa. Die Zugeigenspannungen an der Rohraußenoberfläche erreichen 600 MPa (vgl. Fig. 2).

Beim nachfolgenden Laserschneiden und den Beiz- und Elektropolierprozessen in mineralischen Säuregemischen werden die Druck- und Zugeigenspannungen auf ca. 50 % abgebaut. Der finale Stent hat nunmehr einen Außendurchmesserbereich zwischen 1,78 und 1,79 mm. Das bedeutet, dass von den beiden Rohrwandseiten jeweils 5 µm innen und 10 µm außen abgetragen wurden. In der Summe verringert sich der Außendurchmesser um 20 µm und der Innendurchmesser vergrößert sich um 10 µm gegenüber dem innendruckbeaufschlagtem Rohr. Die verbleibenden Druckeigenspannungen der Innenoberflächen des nunmehr vorhandenen Stents betragen nunmehr noch zwischen 250 und 300 MPa. Die daraus resultierende erhöhte Wechselfestigkeit erhöht die Grenzlastspielzahl um 25 % gegenüber Stents aus unbehandeltem Rohr

Fig. 2 stellt schematisch den Verlauf der Eigenspannungen von der Rohrinnenseite zur Rohraußenseite dar.

Dabei bedeuten die Bezugszeichen
- 1: Rohrinnenseite
- 2: Rohraußenseite
- 3: Rohrwandmitte
- 4a: Skalenwert für die maximale Druckspannung an der Innenseite des innendruckbeaufschlagten Rohres
- 4b: Skalenwert für die maximale Druckspannung an der Stentinnenseite
- 5a: Skalenwert für die maximale Zugspannung an der Außenseite des innendruckbeaufschlagten Rohres
- 5b: Skalenwert für die maximale Zugspannung an der Stentaußenseite
- 6: Druckspannungsverlauf
- 7: Zugspannungsverlauf

Nach der Innendruckbeaufschlagung ergibt sich der durch die durchgezogene Linie dargestellte Spannungsverlauf, der eine initiale maximale Druckspannung von 500 MPa auf der Rohrinnenseite und eine initiale maximale Zugspannung 500 MPa auf der Rohraußenseite entstehen lässt. Die entstehenden Flächenintegrale ober- und unterhalb der spannungsfreien Nulllinie sind gleich groß.

Durch den bei der Stentherstellung realisierten Masseabtrag und die dadurch eintretende Reduzierung der Wanddicken wird auch das Eigenspannungsniveau reduziert. Der gestrichelte Verlauf der Fig. 2 stellt den Eigenspannungszustand von der Steginnenseite zur Stegaußenseite dar. Prinzipiell zeigt sich eine Reduzierung des jeweiligen Eigenspannungsniveaus auf bzw. um 50 % vom Zustand des innendruckbeaufschlagten Rohres.

## Patentansprüche

1. Verfahren zur Erhöhung der Wechselfestigkeit eines röhrenförmigen Halbzeuges für ein medizinisches Implantat, umfassend die Schritte:
a) Bereitstellen des röhrenförmigen Halbzeuges und
b) Beaufschlagen des röhrenförmigen Halbzeuges von innen mit Druck, so dass eine plastische Verformung des Außenumfangs des rohrförmigen Halbzeuges um mindestens 0,2 % erfolgt.

2. Verfahren nach Anspruch 1, wobei das röhrenförmige Halbzeug aus Metall oder einer Metalllegierung besteht.

3. Verfahren nach einem der Ansprüche 1 oder 2, wobei das Halbzeug ein Halbzeug für einen Stent oder eine Herzklappe ist.

4. Verfahren nach einem der vorangehenden Ansprüche, wobei die Druckbeaufschlagung mittels einer Flüssigkeit erfolgt.

5. Verfahren nach Anspruch 4, wobei es zu einer Auflegierung von Anteilen der Flüssigkeit in die Innenoberfläche des Halbzeugs kommt.

6. Verfahren nach Anspruch 4 oder 5, wobei die Druckbeaufschlagung mittels niedrig schmelzender Metalle oder Metalllegierungen, bevorzugt flüssiges Sn, Zn, In, Ga, Li, Na, Bi und deren Legierungen, erfolgt.

7. Verfahren nach Anspruch 5 oder 6, wobei eine Auflegierung bis maximal 50% der Tiefe des Halbzeugquerschnittes erfolgt.

8. Röhrenförmiges Halbzeug für ein medizinisches Implantat hergestellt nach einem Verfahren nach den Ansprüchen 1 bis 7, wobei an der Innenoberfläche Druckeigenspannungen vorliegen, die ca. 15 %, bevorzugt 20 %, weiter bevorzugt 30 % und besonders bevorzugt 40 %, der bei Raumtemperatur im Zugversuch ermittelten Dehngrenze Rp 0,2 betragen und/oder ≥ 20 MPa sind.

9. Röhrenförmiges Halbzeug für ein medizinisches Implantat nach Anspruch 8, umfassend auf seiner Innenseite eine Auflegierung.

10. Röhrenförmiges Halbzeug für ein medizinisches Implantat nach Anspruch 8 oder 9, hergestellt oder herstellbar nach einem Verfahren nach einem der Ansprüche 1 bis 8.

11. Medizinisches Implantat, hergestellt aus einem Halbzeug nach einem der Ansprüche 8 bis 10.

12. Medizinisches Implantat nach Anspruch 11, ausgewählt aus der Gruppe bestehend aus Stent und Herzklappe.

## Claims

1. A method for increasing the fatigue strength of a tubular semifinished product for a medical implant, the method comprising the following steps:
a) providing the tubular semifinished product; and
b) applying pressure internally to the tubular semifinished product such that the outer circumference of the tubular semifinished product is subject to plastic deformation by at least 0.2%.

2. The method according to claim 1, wherein the tubular semifinished product consists of metal or a metal alloy.

3. The method according to either one of claims 1 or 2, wherein the semifinished product is a semifinished product for a stent or a cardiac valve.

4. The method according to any one of the preceding claims, wherein the pressure is applied by means of a liquid.

5. The method according to claim 4, wherein fractions of the liquid are additionally alloyed into the inner surface of the semifinished product.

6. The method according to claim 4 or 5, wherein the pressure is applied by means of metals or metal alloys having a low melting point, preferably liquid Sn, Zn, In, Ga, Li, Na, Bi and alloys thereof.

7. The method according to claim 5 or 6, wherein an additional alloying occurs up to at most 50% of the depth of the cross-section of the semifinished product.

8. A tubular semifinished product for a medical implant produced by a method according to claims 1 to 7, wherein internal compressive stresses are present at the inner surface which are approximately 15%, preferably 20%, more preferably 30%, and particularly preferably 40%, of the proof stress Rp 0.2 established at room temperature in the tensile test and/or are ≥ 20 MPa.

9. The tubular semifinished product for a medical implant according to claim 8, comprising an additional alloying on its inner face.

10. The tubular semifinished product for a medical implant according to claim 8 or 9, produced or producible by a method according to any one of claims 1 to 8.

11. A medical implant produced from a semifinished product according to any one of claims 8 to 10.

12. The medical implant according to claim 11, selected from the group consisting of a stent and heart valve.

## Revendications

1. Procédé d'augmentation de la résistance à la fatigue d'un produit semi-fini en forme de tube destiné à un implant médical, comprenant les étapes :
a) de fourniture du produit semi-fini en forme de tube, et
b) de sollicitation du produit semi-fini en forme de tube à partir de l'intérieur avec une pression de sorte qu'il se produit une déformation plastique de la circonférence extérieure du produit semi-fini en forme de tube d'au moins 0,2 %.

2. Procédé selon la revendication 1, dans lequel le produit semi-fini en forme de tube est constitué de métal ou d'un alliage métallique.

3. Procédé selon l'une des revendications 1 ou 2, dans lequel le produit semi-fini est un produit semi-fini pour un stent ou une valve cardiaque.

4. Procédé selon l'une des revendications précédentes, dans lequel la sollicitation en pression a lieu au moyen d'un liquide.

5. Procédé selon la revendication 4, dans lequel il s'agit d'un dépôt d'alliage de parties de liquide dans la surface intérieure du produit semi-fini.

6. Procédé selon la revendication 4 ou la revendication 5, dans lequel la sollicitation en pression a lieu au moyen de métaux ou d'alliages métalliques à points de fusion faibles, de préférence du Sn, Zn, In, Ga, Li, Na, Bi liquide ou de leurs alliages.

7. Procédé selon la revendication 5 ou la revendication 6, dans lequel il y a un dépôt d'alliage jusqu'à un maximum de 50 % de la profondeur de la section transversale du produit semi-fini.

8. Produit semi-fini en forme de tube destiné à un implant médical fabriqué d'après un procédé selon l'une des revendications 1 à 7, dans lequel des contraintes internes de compression se produisent sur la surface intérieure qui sont d'environ 15 %, de préférence de 20 %, plus préférentiellement de 30 % et de manière particulièrement préférée de 40 % à la température ambiante dans la limite élastique Rp de 0,2 déterminée dans l'essai de traction et/ou ≥ 20 MPa.

9. Produit semi-fini en forme de tube destiné à un implant médical selon la revendication 8, comprenant un dépôt d'alliage sur sa face intérieure.

10. Produit semi-fini en forme de tube destiné à un implant médical selon la revendication 8 ou la revendication 9, fabriqué ou pouvant être fabriqué d'après un procédé selon l'une des revendications 1 à 8.

11. Implant médical fabriqué à partir d'un produit semi-fini selon l'une des revendications 8 à 10.

12. Implant médical selon la revendication 11, choisi dans le groupe constitué d'un stent ou d'une valve cardiaque.
